# EUROPEAN PATENT APPLICATION

(11) **EP 1 393 719 A1**
(43) Date of publication of application: **03.03.2004**
(21) Application number: 02018907.2
(22) Date of filing: 23.08.2002
(51) Int. Cl.: A61K 9/127, A61K 31/47

(54) **Camptothecin-carboxylate formulations**

(71) Applicant: Munich Biotech AG, 82061 Neuried (DE)
(72) Inventor: The designation of the inventor has not yet been filed
(74) Representative: Weiss, Wolfgang, Dipl.-Chem. Dr.

(57) **Abstract**

A composition comprising the carboxylate form of a camptothecin drug or a derivative thereof associated with at least one organic cationic molecule which has a positive net charge cationic molecule), wherein said composition has a molar ratio of at least about 1:1 of an organic cationic molecule to camptothecin carboxylate and is substantially free of the lactone form of said drug or a derivative thereof.

## Description

### 1. Background of the Invention

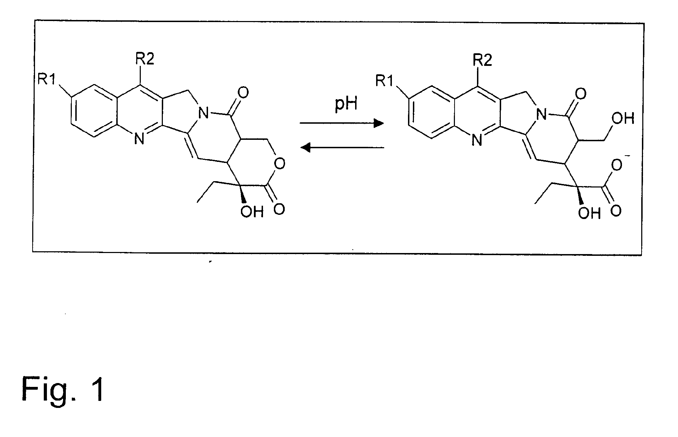

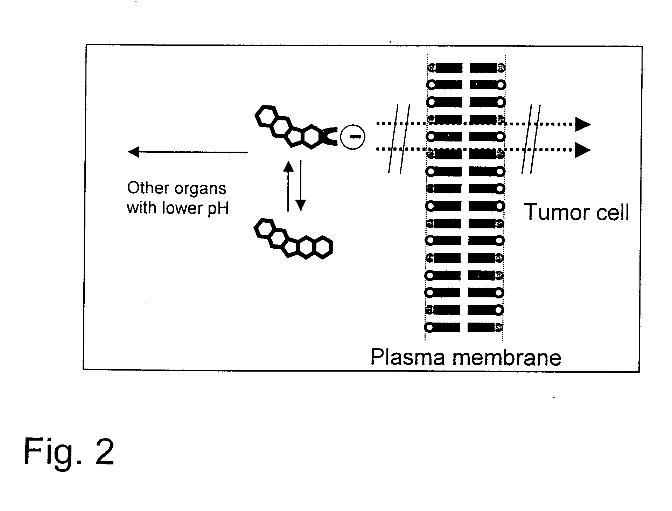

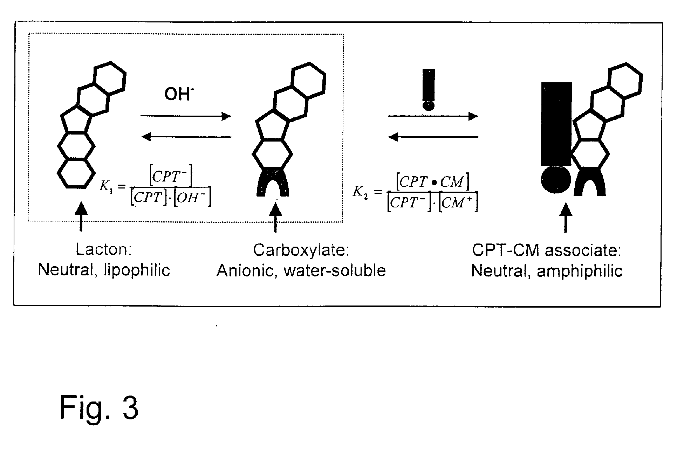

Camptothecin (CPT), is a quinoline-based alkaloid, which can be isolated from the Chinese tree Camptotheca acuminata. It was first described and tested as an anti-cancer drug in the 60ies and 70ies. Anti-tumor activity was noted in animal models and in clinical studies. However, patients experienced severe side reactions such as neutropenia, thrombocytopenia, haemorrhagic cystitis (Wall, M. E. and M. C. Wani (1996). "Camptothecin and taxol: from discovery to clinic." J Ethnopharmacol **51**(1-3): 239-53). It continued to be of high interest as a potential candidate for the development of an anti-cancer drug, and it was found that it has a particular mode of action, wherein binding to the topoisomerase I-DNA complex induces DNA breaks and cell death (topoisomerase I inhibitor).
A fundamental molecular property of CPT is its pH dependent equilibrium between the lactone and the carboxylate form (Fig. 1). The lactone form is lipophilic, while the carboxylate, which predominates at physiological pH and above, is water-soluble. Since the lactone form is too lipophilic to be administered without difficulties, initially, CPT was transformed into the water-soluble sodium salt (NCS 100880). However, due to unacceptable side reactions, the development of that compound was not further pursued.
In subsequent research, the equilibrium between the lactone form and the carboxylate form was found fundamental for the cytostatic activity and the appearance of side effects within anti-cancer treatment, since CPT-carboxylate was identified as being responsible for the observed side reactions. Furthermore, CPT-carboxylate was considered to be significantly less active than CPT-lactone (Hertzberg, et. al. (1989) "Modification of the hydroxy lactone ringof camptothecin: inhibition of mammalian topoisomerasel and biological activity" Journal of Medical Chemistry, 32, 715). The lower membrane permeability of the carboxylate form compared to that of the lactone, and the shift of the molecular equilibrium as a function of the pH value at the respective local physiological environment were considered to be involved in these activity differences (see Fig. 2).
Due to these futile properties of CPT-carboxylate, further efforts for the development of CPT based drugs were concentrated on the control of the equilibrium between the lactone and the carboxylate form. This is especially important, since under physiological conditions, the carboxylate is the predominant molecular form of CPT, and the lactone form hydrolyses to the carboxylate within a short period of time (typically few hours or less). Therefore, a main objective of the development of CPT drugs was to stabilize the lactone form and to find way to administer it without difficulties.
Various strategies have been followed to stabilize the lactone ring and to concomitantly improve the solubility properties of the molecule in order to provide easier administration. Particularly, functionalization of the original molecule to different types of derivates, synthesis of prodrugs, and several types of administration have been pursued (Kehrer, D. F., O. Soepenberg, et al. (2001). "Modulation of camptothecin analogs in the treatment of cancer: a review." Anticancer Drugs **12**(2): 89-105.). However, none of them have brought the desired results of resolving the above described inherent difficulties of camptothecin as an anti-cancer drug.
In another approach, liposomes were used to protect CPT-lactone from hydrolysis. This was realized by encapsulation of CPT in a liposome under acidic conditions, or by embedding CPT into the lipid bilayer of a liposome in order to protect the lactone form from hydrolysis and from blood and serum interactions. In fact, by embedding CPT-lactone in the hydrophobic region of the vesicular lipid bilayer (US 5552156) the lactone form was not exposed to the aqueous environment and hydrolysis was significantly slowed down. However, only very low drug/lipid ratios could be achieved and therefore the necessary dosages for clinical use could not be realized.
In a further liposome-based approach, CPT-lactone was embedded into the lipid bilayer of a liposome comprising phospholipids, which contain unsaturated fatty acids (US 5834012). Thereby a stabilization effect was claimed. It was proposed, that the latter was due to the interaction of CPT in the lactone form with the unsaturated fatty acid chains of the lipids. However, as with other approaches, up to now no substantial breakthrough towards a functional CPT drug formulation could be achieved.
Thus, the problem underlying the present invention was to provide a pharmaceutically active composition of a camptothecin drug and a formulation thereof for most efficient delivery to a subject in need under reduction of side effects.
The solution to the above problem is achieved according to the invention by providing the embodiments characterized in the claims. The invention relates to a composition comprising the carboxylate form of a camptothecin drug or a derivative thereof associated with at least one organic cationic molecule (fig. 3) , preferred an amphiphile or a polymer, which has a positive net charge (cationic molecule), wherein said composition has a molar ratio of the charged groups of at least about 2:1, preferably about 1.5:1, more preferably about 1.1:1 and most preferably about 1:1 of said organic cationic molecule to the carboxylate form of a camptothecin drug, and wherein said composition is substantially free of the lactone form of said camptothecin drug or a derivative thereof.

### 2. Summary of the invention

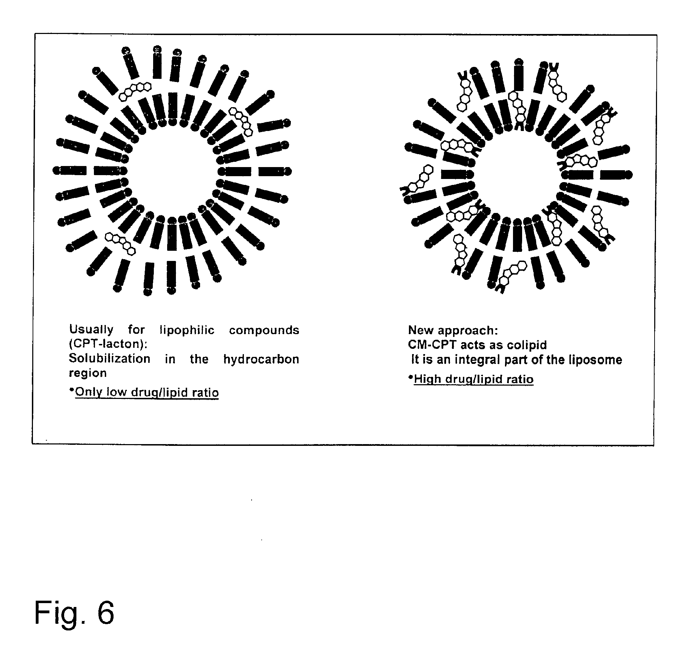

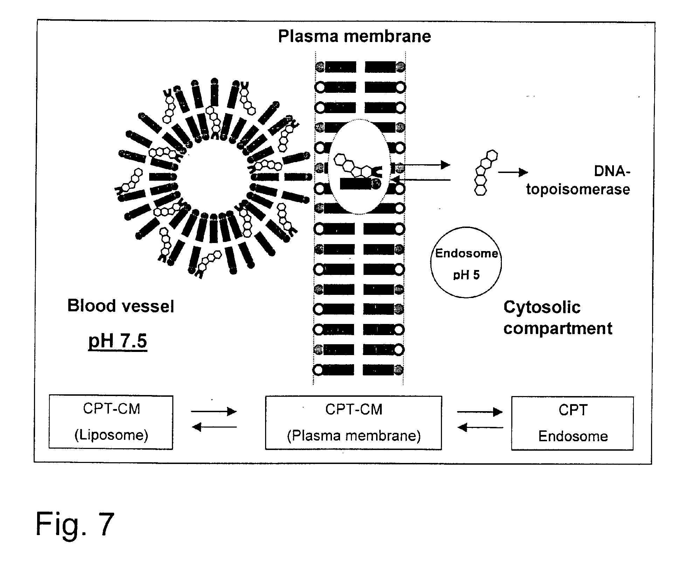

The present invention follows a strategy, which is contrary to the development of CPT drugs within the last 30 years, since it bases on using the carboxylate form instead of the lactone from of a CPT drug. The inventive composition is obtained from CPT-carboxylate and a cationic organic counter molecule (fig. 3), which can be an amphiphilic low molecular weight compound, or a polymer. Subsequently, for the composition, the abbreviation CM-CPT (cationic molecule-CPT), will be used, independently if the organic counter molecule is a low molecular weight amphiphile or a polymer.
The composition is characterized by its physico-chemical properties, which are clearly different to those of the constituting molecules. It can be identified as an independent molecular form in contrast to the carboxylate and the lactone form for example by spectroscopic measurements (fig. 4, fig.5). In contrary to the lactone form of CPT, which is lipophilic, and the carboxylate form, which is hydrophilic, the properties of CM-CPT are determined by the organic counter molecule, i.e., it may be amphiphilic and it may be used, for the assembly of organized molecular nanoaggregates (fig. 6). Unexpectedly, the inventive composition turned out to be useful for producing a pharmaceutical preparation, since, it maintains the cytostatic activity of CPT while it overcomes the disadvantages of the carboxylate as well as those of the lactone form.
The advantages of the present invention are as follows:.
- With the inventive CPT-lipid composition, an active formulation is obtained, which does not have the disadvantages of the CPT drugs so far. The inherent difficulties of the lactone (low solubility and stability in aqueous environment) and the carboxylate form (low membrane permeability, low activity and induction of side reactions) are overcome. With CM-CPT, an active anti-cancer drug is provided, which does not show the side reactions as induced by the CPT-carboxylate.
- It can be brought into aqueous environment and stable colloidal nanoaggregates can be formed. Liposomes with high drug/lipid ratios are obtained (Fig. 6). The release of the free CPT-carboxylate and therefore side reactions are reduced.
- Due to the efficient binding to the cationic counter molecule and the embedding into a lipid matrix (such as a liposomal bilayer), CM-CPT is protected against blood/serum interactions and the deactivation of the CPT as induced by these interactions is suppressed.
- Since the amphiphilic ion pair with the organic cationic molecule has amphiphilic properties, its membrane solubility is increased and cellular uptake of the drug is facilitated / enabled (Fig. 7). Different uptake mechanisms are possible. In addition to the so-called endocytotic pathway, fusion of liposome carrier with the cell membrane and concomitant direct of the release of the complex into the cytoplasma or direct permeation of the complex through the plasma membrane into the cytoplasm.

For the formation of the composition, the carboxylate form is necessary. For most efficient preparation, the camptothecin drug is provided as a camptothecin carboxylate salt. In a preferred embodiment of the present invention, the camptothecin carboxylate is selected from the ammonium, sodium or potassium salt of a camptothecin drug or a derivative thereof. Two preferred procedures for the formation of the salt are:
i) the lactone is dissolved in the suitable alkaline environment, and then the solution is brought to the desired pH or
ii) the lactone is dissolved in ammonia of suitable concentration, and then the solvent is evaporated. Following this procedure, the pure CPT-ammonium salt, without other additives is obtained.

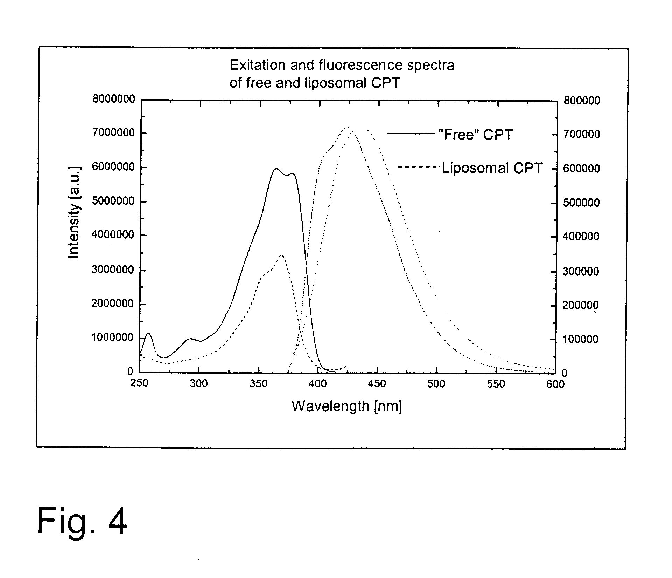

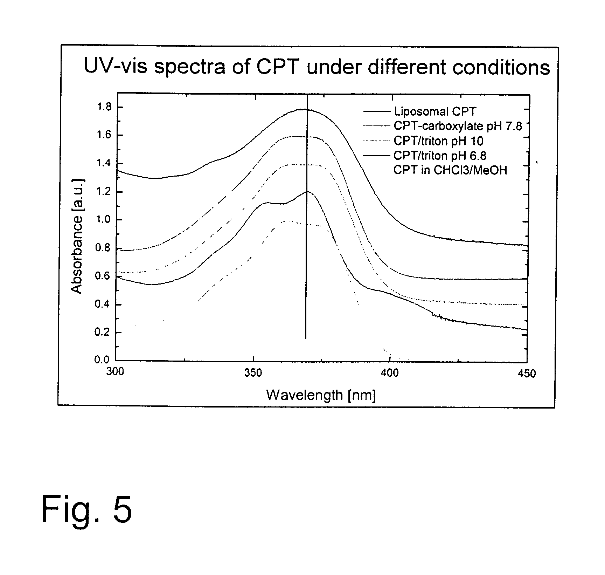

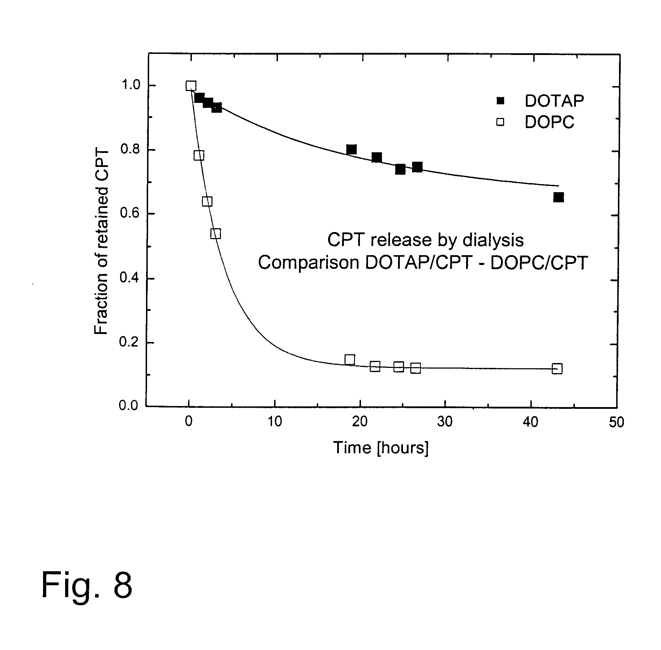

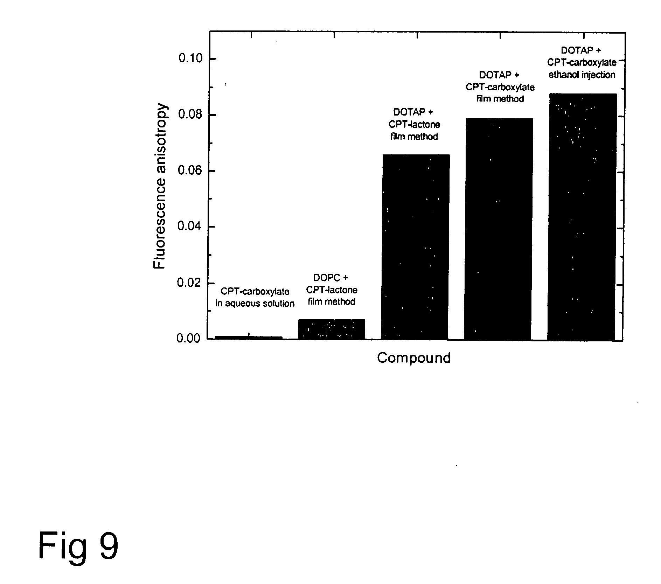

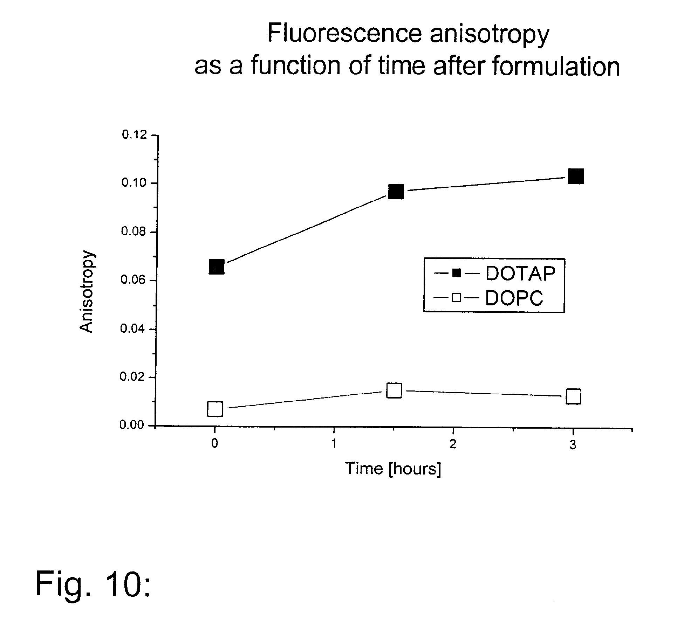

The inventive composition is characterized in that the carboxylate form of a camptothecin drug is associated with an organic cationic molecule as driven by favourable electrostatic forces and amphiphilic interactions. Preferably, the organic counter molecule is a cationic amphiphile or a positively charged polymer. In the text, for both cases, the term CM-CPT is being used to denominate the composition from camptothecin and the organic counter molecule. The composition is obtained by adding the CPT-carboxylate to the counter ion, or vice-versa, in a suitable way. Detailed procedures for the creation of the composition are given in the subsequent sections. The so formed CM-CPT is a new entity, constituting a new molecular composition with unique physico-chemical properties. The presence of the CM-CPT can be determined in situ, for example by spectroscopic methods, because the spectra of the CPT-carboxylate, the lactone and CM-CPT are clearly different (fig.4, fig.5). In fig. 4, the excitation and the fluorescence spectra of the CPT-lactone in organic solvent (CHCl₃/MeOH) and CM-CPT in a liposomal formulation are depicted. CM-CPT displays spectra with different shapes and maximum positions with respect to the free CPT. In fig. 5, the different shapes of the CM-CPT (top), CPT as the carboxylate (second, third) and the CPT as a lactone in two different environments (fourth, fifth) can be made out.
The binding selectivity of CPT to cationic molecules is clearly demonstrated in fig. 8, where the binding of CPT to DOPC (neutral) and to DOTAP (cationic) in aqueous suspensions are compared. Ethanol injections of the lipid solutions into aqueous solutions of CPT-carboxylate were performed, and the resulting colloidal suspensions were dialyzed against a large excess of buffer in order to remove the remaining free CPT-carboxylate (experimental details are given in the following section). The release of CPT-carboxylate as a function of time was determined by UV-vis spectroscopy. As can be seen, for DOPC, virtually all of the CPT was released from the suspension, i.e., no binding to the DOPC was determined. On the contrary, with DOTAP, an equilibrium was reached where the majority of the CPT remained binding to the lipid.
These finding are corroborated as well by fluorescence anisotropy measurements of the CPT. In this experiment, an increase of the fluorescence anisotropy is expected if the fluorophore is inserted into a liposome. As can be seen in fig. 9, with DOPC only a very small anisotropy, close to the one of the free CPT-carboxylate in aqueous solution, was measured, while with DOTAP, for all shown formulations, a much higher anisotropy was observed. These observations indicate, that only with cationic amphiphiles binding of CPT-carboxylate and formation of stable colloidal suspensions occurs. With DOPC, such a binding could not be observed. DOTAP and DOPC both comprise unsaturated fatty acid chains, but DOTAP is positively charged while DOPC is neutral. Therefore, the observations indicate, that the driving force for the binding of CPT-carboxylate is the positive charge of the lipid. In contrast, such as shown in fig. 10, the binding efficacy of CPT-lactone to DOPC is only relatively low. This demonstrates, that the electrostatic interaction driven binding of CPT-carboxylate to cationic molecules is much more efficient that the hydrophobic interaction driven binding of CPT-lactone to the unsaturated fatty acid residues of lipids, which is described in US 5834012. The experimental results are described to more detail in the subsequent section.
The organic cationic molecule used for the present invention can be a cationic amphiphile or a polymer. The polymer can be any polyelectrolyte, such as polyallylamine or polyethyleneimine, as well as polyamino acids or polymeric sugars. The molecular weight is preferably between about 5 and 500 kDa. The CPT binds to the polymer by simple mixing. The resulting composition can be used as a constituent for the self assembly of different types of nanoaggregates (nanoparticles, nanocapsules).
The cationic amphiphile can be monovalent or polyvalent. It is selected from lipids, lysolipids or pegylated lipids with a positive net charge. Useful cationic lipids thereby include:
DDAB, dimethyldioctadecyl ammonium bromide; N-[1-(2,3-dioleoyloxy)propyl]-N,N,N-trimethyl ammonium methylsulfate (DOTAP); 1,2-diacyloxy-3-trimethylammonium propanes, (including but not limited to: dioleoyl, dimyristoyl, dilauroyl, dipalmitoyl and distearoyl; also two different acyl chain can be linked to the glycerol backbone); N-[1-(2,3-dioloyloxy)propyl]-N,N-dimethyl amine (DODAP); 1,2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS);, 3β-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propanaminium trifluoro-acetate (DOSPA); β-alanyl cholesterol; cetyl trimethyl ammonium bromide (CTAB); diC14-amidine; N-tert-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propylamide (DOSPER); N,N,N',N'-tetramethyl-N,N'-bis(2-hydroxylethyl)-2,3-dioleoyloxy-1,4-butanediammonium iodide; 1-[2-(acyloxy)ethyl]2-alkyl(alkenyl)-3-(2-hydroxyethyl)-imidazolinium chloride derivatives as described by Solodin et at. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain a hydroxyalkyl moiety on the quaternary amine, as described e.g. by Feigner et al. [Feigner et al. *J*. *Biol. Chem.* 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropyl ammonium bromide (DORIE-HP), 1,2-dioleyloxypropyl-3-dimethyl-hydroxybutyl ammonium bromide (DORIE-HB), 1,2-dioleyloxypropyl-3-dimethyl-hydroxypentyl ammonium bromide (DORIE-Hpe), 1,2-dimyristyloxypropyl-3-dimethyl-hydroxylethyl ammonium bromide (DMRIE), 1,2-dipalmityloxypropyl-3-dimethyl-hydroxyethyl ammonium bromide (DPRIE), 1,2-disteryloxypropyl-3-dimethyl-hydroxyethyl ammonium bromide (DSRIE); cationic esters of acyl carnitines as reported by Santaniello et al. [US5498633].

In a preferred embodiment the cationic amphiphile is selected from a quaternary ammonium salt such as N-[1-(2,3-diacyloxy)propyl]-N,N,N-trimethyl ammonium, wherein a pharmaceutically acceptable counter anion of the quaternary amino compound is selected from the group consisting of chloride, bromide, fluoride, iodide, nitrate, sulfate, methyl sulfate, phosphate, acetate, benzoate, citrate, glutamate or lactate.

The inventive composition can further comprise amphiphiles with a negative and/or neutral net charge (anionic and/or neutral amphiphile). These can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE, a large family of derivatives is available form Avanti Polar Lipids), 1,2-diacyl-glycero-3-phosphocholines (a large family of derivatives is available form Avanti Polar Lipids), sphingomyelin. The fatty acids linked to the glycerol backbone are not limited to a specific length or number of double bonds. Phospholipids may also have two different fatty acids. In a preferred embodiment the neutral amphiphile is diacylphosphatidylcholine. It is well known, that, in aqueous environment, amphiphiles and as well the polymers mentioned herein can form various types of colloidal nanoparticulate systems. These include suspensions of micelles, liposomes, nanocapsules, any other type of nanoparticles or as well emulsions and
gels. The physico-chemical properties of the described novel composition of this invention are determined as well by the constituting amphiphile or polymer. Therefore, stable suspensions of nanoaggregates can be made up from the composition in an analogous way as for the constituting amphiphile and/or polymer.

Thus, a further aspect of the present invention relates to a colloidal nanoaggregate comprising the inventive composition and having an overall positive charge. In this context overall positive charge means an excess of positively charged molecules in the molecular surface layer of the colloidal nanoaggregate. In a preferred embodiment, the inventive nanoaggregate comprises an excess of cationic lipids of at least 20 mol%, preferably at least 30 mol% and most preferably at least 40 mol%. As an example, if the surface of the nanoaggregate contains 10 mol% negatively charged lipids or negatively charged CPT drug, the amount of positively charged lipid has to be at least 30 %, preferably at least 40 mol% and most preferably at least 50 mol% in order to fulfil these charge requirements. Measurements of the zeta potential can be used to proof the net charge of the colloidal nanoaggregates. Preferably, zeta potentials are in the range between 25 mV and 100 mV, more preferably between 35 mV and 70 mV, depending on the composition (measured in about 0.05 mM KCI solution at about pH 7.5 at room temperature are useful in the context of the present patent. However, zeta potential values lower than 25 mV can be determined when colloidal nanoaggregates were formulated with pegylated lipids.
The inventive nanoaggregate can be present as different supramolecular assemblies with different size ranges. Thus, in further preferred embodiments, the inventive nanoaggregate is an emulsion droplet, a liposome, a micelle, a nanoparticle or a nanocapsule. The particle diameter may range from about 5 nm to about 1000 nm, preferably between 25 nm and 500 nm and more preferably between 100 to 300 nm.
The proportion of a CPT drug in the cationic colloidal nanoaggregates of the present invention is less than about 50 mol%. In some embodiments, the nanoaggregate comprise a camptothecin drug in a proportion from about 1 to 50 mol% and in other embodiments from about 5 to 30 mol%. ln other embodiments, a camptothecin drug is present in about 5 to 15 mol%..

Preferred nanoaggregates of the present invention are cationic liposomes comprising DOTAP, DODAP, analogues of DOTAP or DODAP or any other cationic lipid in an amount, that the net excess of positively charged moieties is at least 20%, preferably of at least 30mol%, more preferably of at least 40%, and most preferably of at least 50% with respect to the total number of molecules. The liposomes may contain further DOPC, pegylated lipids or any other lipid that has a neutral or negative net charge. The liposomes may contain as well pegylated lipids, preferably up to a fraction of 10%, more preferably up to 5%. In case the liposomes do not contain pegylated lipids, the zeta potential of this liposomes is preferably above 30 mV, more preferably above 40 mV, and most preferably above 50 mV. If pegylated lipids are present or any other lipid, the zeta potential of the preferred formulation as measured under the conditions given above can be as well be lower, i.e. in the range close to zero.

It is a further object of the present invention to provide a cationic colloidal nanoaggregate such as liposomes which can be freeze-dried (lyophilized), stored for an extended time period and then reconstituted by adding the removed amount of water to the dry lyophilisates. Reconstitution of the lyophilisates is performed when and where they are to be used without losing a substantial portion of their contents during the freeze-drying process (lyophilization), storage and reconstitution step. To achieve the latter, one or more protective agents such as cryoprotectants can be present. Thus, preferably the inventive nanoaggregate comprises a cryoprotectant wherein the cryoprotectant is selected from a sugar or an alcohol or a combination thereof. Preferably, the cryoprotectant is selected from trehalose, maltose, sucrose, glucose, lactose, dextran, mannitol or sorbitol.

It is a further object of the present invention to provide a concentrated lipid and CM-CPT drug suspension or gel. The concentration is preferably higher that 100 mM, but it can be as well at 200 mM, 500 mM or higher. The concentrate may be stored as it is, or freezing or lyophilization may be applied. One or more protective agents such as cryoprotectants, buffers or other additives can be present. The concentrate can be reconstituted with an aqueous phase which can contain buffers, and other additives in order to obtain a more diluted liposome suspension.

In accordance with other aspects of the invention, a pharmaceutical preparation comprising a pharmaceutically effective amount of the inventive composition or an inventive colloidal nanoaggregate together with a pharmaceutically acceptable carrier, diluent and/or adjuvant is provided.

A further aspect of the present invention relates to a method of producing the inventive nanoaggreagates. Colloidal nanoaggregates can be formed by various methodologies, well known to ordinarily skilled artisans. Sonication, vortexing, mechanical stirring, static mixing, homogenization, injection, microfluidization, colloid mills and/or pressure emulsifiers can be used to prepare nanoaggregates of various types including various orders of addition of materials. The nanoaggregates of the present invention can be formed by a homogenization, a lipid film, a solvent mixing or by a solvent injection procedure.
The nanoaggregates of the present invention can be produced by a method comprising the following steps:
a) providing a camptothecin drug, preferably as a camptothecin carboxylate salt and
b) associating said derivative with a cationic amphiphile which has a positive net charge (cationic amphiphile) and
c) optionally admixing at least one further amphiphile in step b) which has a negative and/or neutral net charge (anionic and/or neutral amphiphile) and
d) forming a colloidal nanoaggregate with an overall cationic charge as described above.

The solvent injection procedure is performed wherein
a) an organic solution comprising at least one cationic amphiphile and optionally at least one anionic and/or neutral amphiphile is added to an aqueous solution of the carboxylate form of a camptothecin drug or a derivative thereof or
b) an organic solution comprising at least one cationic amphiphile and optionally at least one more cationic, anionic and/or neutral amphiphile and the camptothecin drug or a derivative thereof is added to an aqueous solution,
wherein said aqueous solution has a pH value between 3 and 9, preferably between 5 and 8.

The solvent mixing procedure is performed wherein an aqueous solution and a non-aqueous solution are mixed, where the solutions can contain cationic amphiphiles, anionic amphiphiles neutral amphiphiles, polymers buffers, cryoprotectants and other additives, and at least part of the amphiphiles are dissolved in the non-aqueous phase. Then the non-aqueous solvent and other undesired components are removed by suitable methods, preferably evaporation or dialysis. A colloidal suspension of liposomes or a concentrated gel, consisting of lipid and camptothecin drug is obtained. The gel or suspension can be frozen or lyophilised for storage. It can be reconstituted with suitable aqueous phase to give the desired colloidal nanoaggregates.

Alternatively, in a preferred embodiment a lipid film can be formed by two different methods:
the camptothecin drug can be incorporated either directly by dissolving it in an organic solution comprising at least one cationic amphiphile and optionally at least one anionic and/or neutral amphiphile and the camptothecin drug or by forming a lipid film without the drug first.
In the first case, the lipid film is subsequently taken up by an aqueous solution comprising no drug and in the second case with the camptothecin drug.

In a further preferred embodiment, the organic solution comprises an organic solvent selected from methanol, ethanol, propanol, isopropanol, ethylene glycol, tetrahydrofuran, chloroform or diethylether or a mixture of these solvents.

In a preferred embodiment, the aqueous solution further comprises a cryoprotectant which is selected from a sugar or an alcohol or a combination thereof such as trehalose, maltose, sucrose, glucose, lactose, dextran, mannitol or sorbitol.

In an other preferred embodiment the inventive method can further comprise at least one dialysis and/or at least one homogenization and/or optionally at least one sterile filtration and/or optionally a freeze drying and/or optionally a reconstitution step. Dialysis might be necessary in order to remove free camptothecin drug, or in order to remove non-aqueous solvent or other components from the solution. Since the nanoaggregates as achieved by the above-described method do not necessarily have the desired size distribution, a homogenization step may be performed subsequently. This can be performed by extrusion through a membrane with defined pore size, high pressure homogenization, ultrasound treatment, high speed homogenization, or any other homogenization technique well known in the art.

Angiogenesis associated diseases are dependent on blood supply. The local interruption of the vasculature will produce an avalanche of cell death. The vascular endothelium is in direct contact with the blood. It is contemplated that a variety of diseases can be prevented and treated with the foregoing methods and compositions. ln a preferred embodiment, a composition, a colloidal nanoaggregate or a pharmaceutical formulation as provided by the present invention can be used for preventing and/or treating a disease such as cancer, a variety of inflammatory diseases, diabetic retinopathy, rheumatoid arthritis, inflammation, dermatitis, psoriasis, stomach ulcers, macular degeneration, hematogenous and solid tumors. In a further preferred embodiment, the methods and compositions of the present invention can be applied for producing a medicament for preventing and/or treating solid tumors and their metastases such as bladder, brain, breast, cervical, colorectal, endometrial, head and neck or kidney cancer, leukemia, liver or lung cancer, lymphoma, melanoma, non-small-cell lung, ovarian, pancreatic or prostate cancer.

Aspects of the invention work well in that the cells being targeted are endothelial cells. Thus, a further objective of the present invention is producing a medicament for the selective delivery of a camptothecin drug with antimitotic activity to an angiogenic vascular target site, wherein delivery comprises the steps of
a) providing a membrane permeating composition of a camptothecin drug or a derivative thereof, and/or a colloidal nanoaggregate and/or a pharmaceutical composition of the invention,
b) administering said composition and/or colloidal nanoaggregate and/or pharmaceutical composition to a subject in need thereof,
c) allowing the composition and/or colloidal nanoaggregate and or pharmaceutical composition to associate with the target site and
d) releasing and thereby activating said camptothecin drug or said derivative at the target site.

In a preferred embodiment, in step a) the inventive composition and/or the inventive colloidal nanoaggregate and/or a pharmaceutical composition thereof is provided.

Preferably, step d) comprises endocytosis and/or fusion with the cell membrane and/or passive or active diffusion across the cell membrane, wherein endocytosis comprises releasing said camptothecin into the lysosomal compartment.

### 3. Definitions

All technical and scientific terms used in this specification shall have the same meaning as commonly understood by persons of ordinary skill in the art to which the present invention pertains.

**Active ingredient** refers to an agent that is diagnostically or therapeutically effective.

**Amphiphile** is a molecule, which consists of a water-soluble (hydrophilic) and an oil-soluble (lipophilic) part.

**Associating** a negatively charged compound to a cationic amphiphile means binding of said compound to the cationic organic molecule by electrostatic and/or amphipatic (or amphiphilic) forces. The resulting composition is preferably characterized by a ratio close to 1:1, but also ratios of 1.5:1 or >2:1 may be used depending on the nature of the molecules.

A **camptothecin drug** can comprise camptothecin or a derivative thereof. A camptothecin derivative is obtained from any chemical derivatization of camptothecin. A non-limited list of possible camptothecin compounds is given under: http://dtp.nci.nih.gov as from Aug. 19, 2002. In the sketch of the molecule, the most frequent derivatization sites are outlined as R₁-R₅. ln the table, typical examples for derivatization at the different sites are listed. Any combination of these examples and any other derivatization may be performed. The compound may be present as a hydrochloride. The lactone ring may be seven-membered instead of six-membered.

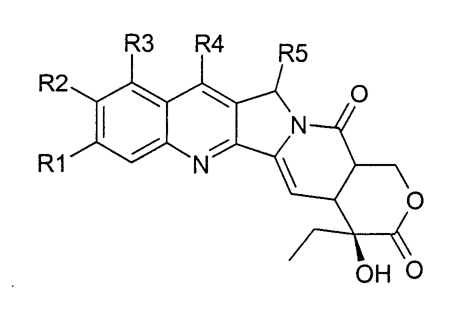

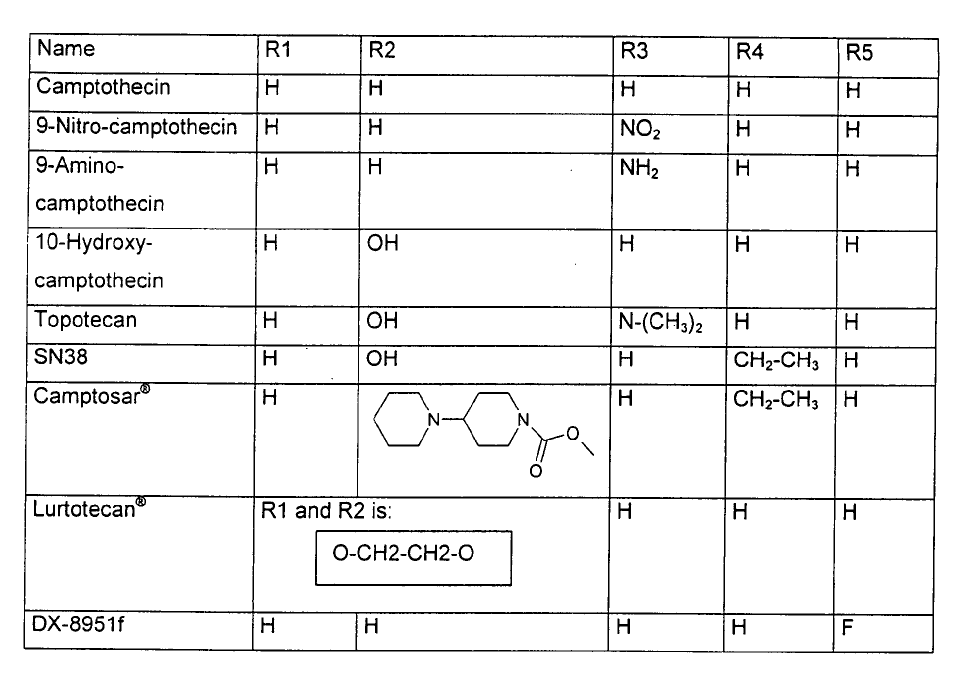

**Cancer** refers to the more common forms of cancers such as bladder cancer, breast cancer, colorectal cancer, endometrial cancer, head and neck cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, hodgkin's disease, leukemia, acute lymphoblastic, leukemia, acute myeloid, liver cancer, medulloblastoma, neuroblastoma, non-hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer.

**Carrier** refers to a diluent, adjuvant, excipient, or vehicle with which a diagnostic or therapeutic agent is administered. The term also refers to a pharmaceutically acceptable component(s) that contains, complexes or is otherwise associated with an active ingredient to facilitate the transport of such an agent to its intended target site. Carriers include those known in the art, such as liposomes, polymers, lipid complexes, serum albumin, antibodies, cyclodextrins and dextrans, chelates, and other supramolecular assemblies.

**Cationic** refers to an agent that has a net positive charge or positive zeta potential under the respective environmental conditions. In the present invention, it is referred to environments where the pH is in the range between 3 and 9, preferably between 5 and 8.

The term "**cationic amphiphile**" or "**cationic lipid**" is used herein to encompass any amphiphile or lipid which has a positive charge. In the present invention, it is referred to environments where the pH is in the range between 3 and 9, preferably between 5 and 8.

**Cationic liposomes** are liposomes which are positively charged. In the present invention, it is referred to environments where the pH is in the range between 3 and 9, preferably between 5 and 8. The cationic liposomes are prepared from the cationic lipids or amphiphiles themselves or in admixture with other amphiphiles, particularly neutral or anionic lipids.

**Colloids** or colloidal particles are particles or molecular aggregates dispersed in a medium in which they are insoluble and have a size between about 5 nm and 5000 nm.

**Colloidal nanoaggregate having an overall positive charge** as used herein refers to a colloidal nanoaggregate comprising optionally the inventive composition and having an excess of positively charged molecules in the outer molecular layer.

**Cryoprotectant** is a substance that helps to protect a species from the effect of freezing.

**Derivative** is a compound derived from some other compound while maintaining its general structural features. Derivates may be obtained for example by chemical functionalization or derivatization.

**Disease characterized by enhanced angiogenic activity** refers to processes such as tissue inflammation, arthritis, asthma, macular degeneration, tumor growth, and diabetic retinopathy.

**Drug** as used herein may be a pharmaceutically acceptable pharmacologically active substance, physiologically active substances and/or substances for diagnosis use.

**Homogenization** refers to a physical process that achieves a uniform distribution between several components. One example is high-pressure homogenisation.

The term **lipid** is used in its conventional sense as a generic term encompassing fats, lipids, alcohol-ethersoluble constitutents of protoplasm, which are insoluble in water. Lipids compose the fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds, and linked to the backbone, the fatty acids could be different (asymmetric), or there may be only 1 fatty acid chain present, e.g., lysolecithins. Mixed formulations are also possible, particularly when the non-cationic lipids are derived from natural sources, such as lecithins (phosphatidylcholines) purified from egg yolk, bovine heart, brain, or liver, or soybean.

**Liposome** is a microscopic spherical membrane-enclosed vesicle (about 50-2000 nm diameter) made artificially in the laboratory. The term "liposome" encompasses any compartment enclosed by a lipid bilayer. Liposomes are also referred to as lipid vesicles. ln order to form a liposome the lipid molecules comprise elongated nonpolar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane. As used in connection with the present invention, the term liposome includes multilamellar liposomes, which generally have a diameter in the range of about 1 to about 10 micrometers and are comprised of anywhere from two to hundreds of concentric lipid bilayers alternating with layers of an aqueous phase, and also includes unilamellar vesicles which are comprised of a single lipid layer and generally have a diameter in the range of about 20 to about 400 nanometers (nm), about 50 to about 300 nm, about 300 to about 400 nm, about 100 to about 200 nm, which vesicles can be produced by subjecting multilamellar liposomes to ultrasound, by extrusion under pressure through membranes having pores of defined size, or by high pressure homogenization.

**Lysolipid** is a lipid where one fatty acid ester has been cleaved resulting in a glycerol backbone bearing one free hydroxyl group.

**Lysophospholipid** is a phospholipid where one fatty acid ester has been cleaved resulting in a glycerol backbone bearing one free hydroxyl group.

**Micelle** is an aggregate of amphiphilic molecules in a colloidal suspension.

**Nanoaggregate** is an assembly on a nanometer to micrometer scale assembled from smaller particles held together by attractive physical forces.

**Nanoparticle** is an entity in the dimensions of nanometers to micrometers.

**Negatively Charged Lipids** are lipids that have a negative net charge. ln the present invention, it is referred to environments where the pH is in the range between 3 and 9, preferably between 5 and 8. Examples are phosphatidic acid, phosphatidylserine, phosphatidylglycerol, phosphatidylinositol (not limited to a specific sugar), fatty acids, sterols.
**Neutral Lipids** are lipids that have a neutral net charge such as cholesterol, 1,2-diacyl-sn-glycero-3-phosphoethanolamine, including but not limited to dioleoyl (DOPE), 1,2-diacyl-glycero-3-phosphocholines, Sphingomyelin. In the present invention, it is referred to environments where the pH is in the range between 3 and 9, preferably between 5 and 8.

**Particle diameter** is the size of a particle. To experimentally determine particle diameters, dynamic light scattering (DLS) measurements, using Malvern Zetasizer 1000 or 3000 (Malvern, Herrenberg, Germany) were performed. For quantitative data analysis (determination of Z(average and PI), in all cases parameters (refractive index, density, viscosity) of pure water were plugged in, even if the aqueous phase contained cryoprotectants to a certain extend. Therefore, for the absolute numbers, a certain systematic deviation with respect to literature data may have to be taken into account.

**Pegylated lipid** is a lipid bearing one ore more polyethylene glycol residues.

**Pharmaceutical composition** is a combination of two or more different materials with superior pharmaceutical properties than are possessed by either component.

Phospholipid is a lipid consisting of a glycerol backbone, a phosphate group and one or more fatty acids wich are bound to the glycerol backbone by ester bonds.

**Positively Charged Lipids** is a synonym for cationic lipids (for definition see definition of "cationic lipids"). In the present invention, it is referred to environments where the pH is in the range between 3 and 9, preferably between 5 and 8.

**Sterol** is a steroid alcohol. Steroids are derived from the compound called cyclopentanoperhydrophenanthrene. Well-known examples of sterols include cholesterol, lanosterol, and phytosterol.

**Therapeutic agent** refers to a species that reduces the extent of the pathology of a disease such as cancer. Such a compound may, for example, reduce primary tumor growth and, preferably, the metastatic potential of a cancer. Alternatively, such a compound may reduce tumor vascularity, for example either by decreasing microvessel size or number or by decreasing the blood vessel density ratio.

**Virtually free** of a species is defined as not detectable by HPTLC.

**Zeta potential** refers to a surface potential of a particle such as a colloidal particle measured with an instrument such as a Zetasizer 3000 using Laser Doppler micro-electrophoresis under the conditions specified. The zeta potential describes the potential at the boundary between bulk solution and the region of hydrodynamic shear or diffuse layer. For the present patent, zeta potential measurements were performed with a Malvern Zetasizer 3000 (Malvern, Herrenberg, Germany).

The following examples should be illustrative only but are not meant to be limiting to the scope of the invention. Other generic and specific configurations will be apparent to those skilled in the art.

### 4. Examples

### 4.1. CPT-carboxylate salt preparation

The carboxylate salt of a CPT drug is obtained by exposing the CPT drug to an alkaline environment with a pH above 9. Explicitly, two procedures comprising the following steps, were applied:
a) The CPT drug is dissolved in 100 mM NaOH to a CPT concentration of 10 mM. The solution may contain as well other additives, such as ions or cryoprotectants. Subsequently, by adding of conc. HCl, the solution is brought to the desired pH. The solution can be diluted, concentrated (for example by solvent evaporation) and further components (ions, buffers) can be added.
b) 2 mg/ml of CPT drug are dissolved in 25% NH₄OH Subsequently the solvent is evaporated. In this way, the pure ammonium salt of the CPT drug-carboxylate is obtained. For further treatment, it can be brought in aqueous phase of a desired pH and selected environmental conditions (buffers, cryoprotectants). Alternatively it can be brought into a suitable organic phase.

### 4.2. CM-CPT synthesis

CM-CPT is obtained from CPT-carboxylate salt by adding a solution of a cationic amphiphile to the carboxylate salt. If desired, subsequently the solvent can be evaporated. The solvent can be Ethanol, Chloroform, Chloroform/Methanol, or any other suitable organic solvent or solvent mixture. Alternatively, the solution of the cationic amphiphile can be added to an aqueous solution of the CPT-carboxylate. In case of a positively charged polymer as a countermolecule, the latter can be present as well in the aqueous phase. For further treatment, the CM-CPT solution or suspension can be used as it was prepared, or the solvent can be removed in order to obtain the pure composition. Subsequently it can be brought into a desired medium.

### 4.3. Liposome formulation

The formulation procedure may consist of the following steps:
1. Providing of CM-CPT and the other liposome forming components
2. Formation of a colloidal suspension by a suitable method
3. (optional) Extrusion through a membrane in order to achieve monolamellar liposomes of uniform size
4. (optional) Dialysis or analogous methods for the separation of non-encapsulated drug and/or components or solvent
5. (optional) A concentration step to achieve a highly concentrated liposomal gel
6. (optionally) Freeze drying
7. (optionally) Freezing
8. (optionally) Reconstitution under suitable environmental conditions

For the formation of the colloidal liposome suspensions, different methodologies can be applied. Subsequently, the most relevant techniques are briefly outlined.

### Film method

Liposomes can be formed by the well known film method, such as described in the literature. Briefly, from the organic solution of the lipid, or the lipid plus the drug, the solvent is evaporated, and a thin film of lipid (or lipid plus drug) is formed at the inner wall of a flask. The thin molecular film is resuspended in an aqueous phase, which can contain further components such as buffers, ions, cryoprotectants and the like. With this procedure, liposomal suspensions are formed in a self-assembly process. A standard formulation is obtained by forming a film of 90 µM DOTAP and 10 µM Camptothecin from a solution in Chloroform/Methanol, 10/1 .The film is then reconstituted with 10 ml of the aqueous phase, in order to achieve a suspension where the total liposomal concentration (lipid + drug) is 10 mM. The aqueous solution contains a cryoprotectant, e. g. glucose or trehalose and buffers (Tris), to achieve a desired pH after reconstitution. A typical pH is in the range between 7 and 8. However, depending on the lipid, as well higher (up to pH 9) or lower (down to pH 5) values can be selected. The pH of the liposomal formulation can be altered after formulation for further treatment. Thus, a liposomal formulation with the drug/lipid ratio of 1:9, and with a total (lipid+drug) concentration of 10 mM is obtained. Other typical molarities are 15 mM, 20 mM or 25 mM. If necessary, molarities up to 50 mM or higher can be formulated. The drug:lipid ratio usually is selected to be in the rage from 5:95 to 20:80. The lipid phase can consist of only cationic lipid, such as DMTAP, DOTAP, DPTAP, DSTAP, DOTMA, or DDAB, or it can consist up to 60% of charged and non-charged colipids. Standard formulations which have been used by us most frequently consist of CPT/DOTAP/DPOC = 5:47.5:47.5, or 5:55:45 or 10:45:45 or 10:60:30. ln table 1 the results form physico-chemical characterization of such liposomal formulations are given. Accordingly to the described procedure, formulations comprising other cationic lipids, such as DMTAP, DSTAP, DDAB, DOTMA and the like can be achieved.
In an alternative variety of the formulation procedure 1a) the camptothecin is transformed into the carboxylate, or the carboxylate salt for formulation. Such a way the CPT is 'activated' for easier association of the drug to the cationic lipid, as, in fact, the carboxylate is the molecular form which associates to the latter. CPT is solubilized in NH₃, and the volume which contains the necessary amount of the drug (i.e., 10 µM in the example described in 1 a) is added to a flask. After evaporation of the solvent a film of the ammonium salt of the CPT-carboxylate is formed. Then the organic solution of the lipid is added and the solvent is evaporated. Thus a camptothecin and lipid film is achieved, where the camptothecin binds more readily to the cationic lipid. Particularly for low pH values, it is easier to obtain liposomes with high drug/lipid values. The subsequent procedure is analogous to the one described above.
ln a further variety, the CPT-carboxylate can be part of the aqueous solution for reconstitution of the pure lipid film. ln that case, a separation step (such as outlined below), or a concentration step (such as below) can be performed after formulation.

### Organic Solution Injection

Liposomal suspensions can be achieved through the injection of a solution of the lipid, or the lipid+drug, into the aqueous solution. A typical solvent for the lipid, or the lipid+drug phase is ethanol ('ethanol injection'). In the present case, a new procedure is employed, in the sense that ethanol injection into the aqueous solution of the camptothecin-carboxylate is performed. As due to the attractive interaction between the drug and the cationic lipid, camptothecin-containing liposomes are formed in a self-assembly process. In order to achieve 10 ml of a 10 mM suspension of CPT/DOTAP 1:9, a 10 µM solution of CPT-carboxylate, optionally containing further additives as outlined in the previous section, is prepared. Preferentially, the ammonium salt of the CPT-carboxylate, Used. Alternatively the CPT-carboxylate can be formed by dissolving CPT at high pH in another base, and subsequently the pH is brought to the desired value by buffers. The aqueous phase can have a pH between 5 and 9, according to the necessities of the experiment. In the examples which are presented here (tab. 2) a pH of 7.5 was selected. The ethanolic lipid solution has a concentration between 200-400 mM. The suitable volume of the lipid solution in ethanol is injected under vigorous stirring. All compositions and concentrations as described in the previous section can be achieved by this approach. As an alternative to ethanol, as well other suitable solvents or mixtures thereof can be taken. Typically, these are alcohols, ethers, chloroform, hydrocarbons, etc. As well solvents in the supercritical state can be applied, such as hydrocarbons, carbon dioxide, perfluorinated compounds, etc. Subsequently to the described formulation procedure, extrusion (2) dialysis (3), a concentration step (4) or freeze drying can be performed.
Alternatively to the injection of the pure lipid solution, both, the lipid(s) and the CM-CPT drug can be dissolved in the injection solution. All other steps are equivalent.

### 4.4. Extrusion

The liposomal suspensions as achieved by the above-described methods do not have necessarily the desired size distribution. Therefore, an extrusion through a membrane of defined pore size can be performed subsequently. In our experiments we have usually performed extrusion through membranes of 200 nm pores size (Osmonics Inc., Poretics, polycarbonate 0.2 Microns). Other typical extrusion membranes were with 100 nm or with 400 nm pore size. Size distributions were controlled by quasi-elastic light scattering (Malvern, Herrenberg, Germany). Examples of liposomal sizes after extrusion are given in tables 1 and 2.

### 4.5. Separation of low molecular compounds

Dialysis techniques were used to separate low molecular components from the liposomal suspensions. This was particularly the case for the experiments with ethanol injection, where in addition to a fraction of non-liposomal camptothecin, a certain amount of ethanol was present in the suspension. Dialysis tubes from regenerated cellulose with a MWCO of 8,000-10,000 (Roth, Karsruhe Germany) were used. Alternatively, the so called "cross-flow" technique (Vivascience, Sartorius Group, Göttingen, Germany) with Polyethersulfone membranes, MWCO 50,000 were used. The release of the free CPT was monitored by UV-vis spectroscopy. The amount of the released CPT-carboxylate depends on the lipid and drug concentration, the drug/lipid ratio, pH and other parameters. An example is given in fig.8, where the results from dialysis of 10 mM liposomes suspensions DOTAP/CPT and DOPC/CPT 90/10 are shown. With a dilution factor of 20, more that 60% of the CPT was retained by the DOTAP liposomes, while with DOPC liposomes, less that 10 % were retained, i.e., virtually all CPT was released.

### 4.6. Freezing and Freeze-drying

Freeze drying of the liposomal formulations was performed using standard equipment and adopted protocols (Epsilon 2-12D, Control Unit LMC-2, Christ, Osterode, Germany). The composition and the physical state of the suspensions were characterized after reconstitution such as described below. The suspension as described above could be frozen and brought back to room temperature, without drastically affecting the aggregation state of the liposomes, such as proven by quasi-elastic light scattering measurements.
Results from size measurements before and after lyophilization/freezing are given in tab. 2.

### 4.7. Concentrated liposome suspensions and gels

Pre-formed liposome suspensions are concentrated by standard techniques such as dialysis against polymer solutions, 'cross-flow', or evaporation of solvent. The concentrated suspensions can be brought back to the previous, or any other concentration, without substantial changes of the original size distribution.
To directly prepare concentrated liposome suspensions and gels, an aqueous phase, containing (optionally) one or more of the components, and an organic phase, containing the other components are mixed. For example, an aqueous solution of 2 mM CPT carboxylate, 0.5% trehalose and 1 % tris/HCl buffer is mixed with a 6 mM solution of DOTAP in ethanol, that the final CPT-carboxylate/DOTAP ratio is 10/90. A homogeneous mixture is obtained. The solvent is the evaporated under reduced pressure at room temperature, until the desired concentration is obtained. It is possible to evaporate only the ethanol (together with the eutectic fraction of water) or, with higher vacuum, as well the water. It is possible to add Ethanol to the semi-concentrated suspension for further evaporation. ln this way, for example, suspension with a concentration of 100 mM, 200 mM and 500 mM can be obtained. After reconstitution with water or buffer to a lower concentration, a liposome suspension, where the size distribution is a function of the previous maximum concentration is obtained. Examples are given in tab. 3.

### 4.8. Cationic polymer bound CPT

CBT binding to cationic polymers is achieved by mixing a solution of CPT-carboxylate and of the polymer. The solutions can be in water and/or in organic solvents, depending on the experimental necessities and the type of the polymer.
A typical type of polymers are polyelectrolytes, such as polyallyamine. Both components are provided in the aqueous phase, containing, optionally buffers, ions and cryoprotectants. The polyelectrolytes are deposited onto oppositely charged surfaces and nanoparticles by physisorption. By repetitive deposition of positively and negatively charged polymer (such as polyallyamine or polyallyamine/CPT and polystyrene sulfonate) organized layered structures are obtained. For the deposition procedure, standard protocols are applied (Radtchenko lL, Sukhorukov GB, Leporatti S, Khomutov GB, Donath E, Mohwald. "Assembly of Alternated Multivalent lon/Polyelectrolyte Layers on Colloidal Particles. Stability of the Multilayers and Encapsulation of Macromolecules into Polyelectrolyte Capsules" J Colloid Interface Sci. 2000 Oct 15;230(2):272-280). With this technique, nanoparticles, comprising CPT/polyelectrolyte, are assembled in a controlled way.

### 5. Physico-chemical characterization

### 5.1. Fundamental aspects of CPT interaction with cationic lipids

For the present invention the interaction between CPT and the cationic organic molecules is fundamental. Therefore, the binding of CPT-carboxylate to lipids was characterized by a variety of methods. Particularly, the binding properties to DOTAP and to DOPC were compared, in order to highlight the advantages of the present invention with respect to other, earlier approaches.
Liposomal suspensions were prepared by ethanol injection of a lipid solutions into aqueous solutions of CPT-carboxylate, and the suspensions were dialized in order to remove free, non-liposomal CPT. The amount of the liposomal and the released CPT were determined by UV-vis spectroscopy. ln fig. 8, the results with DOTAP and DOPC, under identical conditions are shown. The concentration of the CPT which remained in the liposomal suspension is depicted as a function of dialysis time. As can be seen, virtually all of the CPT from the DOPC suspension is released in a short time scale, while for DOTAP liposomes, more that 60 % of the CPT remains with the liposomal suspension. This demonstrates, that only with DOTAP, but not with DOPC an attractive interaction and an effect of liposomal stabilization can be determined for the CPT-carboxylate.
ln order to get further information on the CPT-lipid interaction form an independent method, fluorescence anisotropy measurements were performed. Colloidal suspensions from DOTAP and from DOPC together with CPT in different molecular forms were made up. The fluorescence anisotropy was taken as a measure for the binding strength of CPT to the liposome. In fig. 9 the results of various measurements are shown. As expected, for the CPT-carboxylate solution without lipid, the anisotropy was zero, and as well with DOPC liposomes, the anisotropy was rather small. On the contrary, in all three experiments with DOTAP, the anisotropy was much higher, indicating CPT-carboxylate binding to the liposome. Depending on the mode of preparation, the anisotropy was different directly after formulation, but in all cases the same equilibrium value was reached after few hours. The highest anisotropy was found for the experiment where the liposomes were made by ethanol injection into aqueous solution of CPT, while when the liposomes were made with the CPT-lactone by the film method, it was significantly lower. This indicates, that in fact, the carboxylate form of CPT is necessary for efficient binding to the cationic lipid and for the formation of the CM-CPT containing liposomes.
This conjecture is clearly supported by the data depicted in fig. 10. Liposomes from lipid and CPT-lactone were prepared by the classical film method and the anisotropy was determined at different times after formulation. While for DOPC the anisotropy remains at a low level, with DOPAP it increases within a time scale of three hours to a value almost two times as high as the initial one. This can be interpreted in the sense, that the lactone is transformed into the carboxylate, and only then it efficiently binds and inserts into the liposome.

Summarizing, these data indicate consistently, that for highest binding efficiency of CPT to a lipid, it is necessary to provide the CPT in the carboxylate form, and to use a positively charged lipid as a countermolecule. The resulting CM-CPT can be clearly individualized and characterized by its physico-chemical properties. On the contrary, no such CM-CPT aggregate is formed with anionic or neutral lipids. This holds as well if the latter contain unsaturated fatty acids.

### 5.2. Characterization of the liposomal formulations.

All stages of formulation are monitored by HPLC analysis (lipid +drug) and by UV-vis and fluorescence spectroscopy. ln HPLC analysis a slight loss of material after extrusion can be observed. UV-vis and fluorescence spectroscopy serves as a qualitative means in order to control formulation efficacy.
It is well known, that the spectrum of CPT depends strongly on the molecular state and on the local environment. Therefore, qualitatively, the spectra of the lactone form, the carboxylate and the liposomal CPT can be distinguished from the shape of the spectra (fig. 4, fig. 5). ln Fig. 4 the excitation and emission spectra of the lipid-bound CPT and the free drug in organic solvent (Chloroform/Methanol) are shown. ln the fluorescence spectrum, binding to the lipid induces a significant red-shift and a change of the peak shape. The changes of the absorption spectrum are most clearly seen in Fig. 5. The upper curve gives the spectrum of lipid-bound CPT, and subsequently spectra of free CPT under different conditions are shown. The second and the third curve correspond to the lactone form of the molecule, and the fourth and the fifth curve correspond to the lactone form of the molecule.
Quasi-elastic light scattering (Zetasizer 1000 and Zetasizer 3000, Malvern Instruments, Herrenberg, Germany) was measured in order to determine the size distribution of the liposomes. The zeta potential was determined by a Zetasizer 3000 (Malvern Instruments). In Tab.1, the results from the characterization of some exemplary formulations are shown.

### 6. In vitro determination of the cytostatic potential of LipoCam

The efficacy of a cytostatic drug is determined in vitro by analysing the decrease of cell viability in correlation to the drug concentration. The drug concentration at which cell viability is inhibited to 50 % ("IC₅₀") is used as index for the inhibitory potential of a respective drug. The higher the cytostatic potential, the lower is the IC₅₀ value. Drugs with high inhibitory potential have IC₅₀ values in the nM range. Here we compared 3 formulations of liposomally encapsulated Camptothecin carboxylate (RM544=Lipocam I, RM541=Lipocam II, RM542 = Lipocam lll) with the free carboxylate (RM543).

### Principle:

A suitable cell line (i.e. endothelial or tumor cell line) is seeded at a constant densities in four 24-well plates. After one or two days, a series of 11 consecutive drug dilutions is added to cover the range between 0 - 5000 nM final drug concentration. Each individual concentration is measured in 2 wells independently to increase the accuracy of the assay. Two wells without drug serve as control. The cells are incubated at optimal growth conditions (5-5.5 % CO₂, 37°C, ~ 90% humidity). After 72 h, the cell viability in each well is determined by measuring the activity of mitochondrial dehydrogenases (MTT assay). In viable cells the MTT substrate is converted to a blue, cell impermeable dye (Formazan). After around 1 h, the medium is removed, cells are lysed with isopropanol/0,04% HCl and the amount of the blue Formazan quantitated in an ELISA reader at 550 nm (measured against lysis buffer as blank). The experiment is evaluated using the Sigma Plot analysis software by plotting the mean OD₅₅₀ₙₘ value (of the 2 wells containing the identical drug concentration) against the respective drug concentration. The IC₅₀ concentration is taken at the half-maximal OD₅₅₀ₙₘ value. A high inhibitory potential results in a low IC₅₀ value.

### Experiment:

2x 10⁴/cm² Ea.Hy926 cells (transformed human endothelial line) are seeded into four 24-well plates and cultivated over night. At day 1, a series of eleven 5x concentrated master dilutions of each Camptothecin formulation in cell culture medium is prepared (25000, 10000, 5000, 2500, 1250, 500, 250, 50, 25, 12.5, 5 nM). Having prepared all dilutions, the culture medium is removed from the cells and 400 µl of fresh culture medium + 100 µl of the respective master drug concentration is added (each concentration to 2 wells). This results in a 1:5 dilution of the master series (final drug concentrations between 1 nm and 5000 nM). To two wells no drug is added (controls). The plates are cultivated further 72 h. After this incubation period, the medium is replaced by fresh medium containing the MTT substrat and incubated for 1h. The medium is removed, the cells are lysed in isopropanol/0.04% HCl and the OD at 550 nm is measured.

### Result:

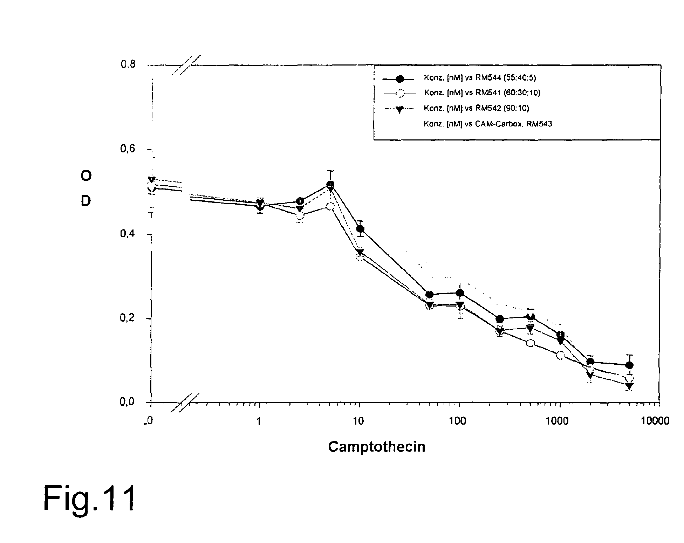

As demonstrated in Fig. 11, all three LipoCam formulations (RM541, RM542, RM544) display a comparable or better growth inhibition curve than free Camptothecin carboxylate. The lC₅₀ values are around 25-30 nM. Therefore, liposomally encapsulated Camptothecin carboxylate has a high cell inhibitory potential in vitro.

### 7. Therapeutic efficacy of LipoCam in A-375 melanoma of nude mice

NMRI-nude mice were purchased from Elevage Janvier and housed in isolated ventilated cages under save environmental conditions (SPF facility, 22°C, 30 - 70% humidity, 12 h light/dark cycle) with food and water *ad libitum*. Experimental design was reviewed and approved by local government.
Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day 0.
Mice were assigned to the experimental groups (8 animals per cage), housed and handled (including monitoring of the body weight gain) at least five days before tumor inoculation (= day - 6 to 0). Drug treatment begins after the tumors reached a volume of approximately 100 mm³. The drugs were given by iv injection, three times a week (Mo, Wed, Fri) for the following three weeks at equitoxic doses. The drugs were prepared as described in example. Equitoxic doses of the compounds were determined in separate experiments, where toxicity was evaluated based on haematological parameters, body weight and animal clinical observations (see tab. 3). The solutions were administered slowly in a volume of -10 µl/g body weight.
Animals were clinically monitored during the whole experiment and for at least one week after treatment was finished. Monitoring of tumor size was performed three times a week after staging, before application during treatment period and during recovery period (at least one week). The tumor dimensions were measured by calliper and the tumor size was calculated according to the following formula: V = π L W² / 6 (L = greatest lengt, W = width of perpendicular axis). The body weight of individual animals was monitored at least twice during handling period (e.g. day -6 and 0), after tumor inoculation, after start of treatment and during recovery period (at least one week) for all groups. EDTA blood was collected from the retrobulbar plexus at four different points: during handling (day -3), tumor staging (day 7), in the middle of treatment (~day 21), and at the end of the recovery period (day 28) from 4 animals of all treatment groups for hematology. The number of red and white blood cells and platelets were determined using an automated cell counter (Abbott Cell Dyn 3500).

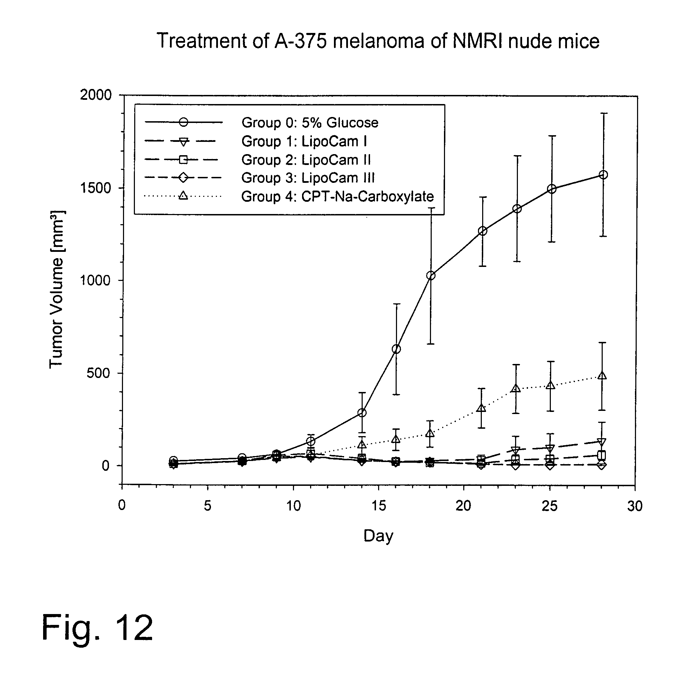

Whereas tumors in the control group showed a rapid and progressive tumor growth, all drug formulations - applied at equitoxic doses - showed an anti-tumor effect (Fig. 12). The most effective formulation was LipoCam lll, with total tumor regression in most animals. LipoCam l and LipoCam II showed also a temporary tumor regression with a slow re-growth of tumors beginning in the second half of the treatment period. CPT-Na-Carboxylate showed only a retardation as compared to the control group.

### 8. Human Therapy Treatment Protocols

This example is concerned with human treatment protocols using the formulations disclosed. Treatment will be of use for diagnosing, preventing and/or treating various human diseases and disorders associated with enhanced angiogenic activity. It is considered to be particularly useful in anti-tumor therapy, for example, in treating patients with solid tumors and hematological malignancies or in therapy against a variety of chronic inflammatory diseases such as psoriasis.

A feature of the invention is that several classes of diseases and/or abnormalities are treated without directly treating the tissue involved in the abnormality e.g., by inhibiting angiogenesis the blood supply to a tumor is cut off and the tumor is killed without directly treating the tumor cells in any manner.

Methods of treating such patients using lipid:drug complexes have already been formulated, for example, see document incorporated herein by reference. It is contemplated that such methods may be straightforwardly adapted for use with the method described herein. As discussed above, other therapeutic agents could be administered either simultaneously or at distinct times. One may therefore employ either a pre-mixed pharmacological composition or "cocktail" of the therapeutic agents, or alternatively, employ distinct aliquots of the agents from separate containers.

The various elements of conducting a clinical trial, including patient treatment and monitoring, will be known to those of skill in the art in light of the present disclosure.

For regulatory approval purposes, it is contemplated that patients chosen for a study would have failed to respond to at least one course of conventional therapy and would have objectively measurable disease as determined by physical examination, laboratory techniques, or radiographic procedures. Such patients would also have no history of cardiac or renal disease and any chemotherapy should be stopped at least 2 weeks before entry into the study.

Prior to application, the formulations can be reconstituted in an aqueous solution in case the formulation was freeze dried. Again, the required application volume is calculated from the patient's body weight and the dose schedule.

The disclosed formulations may be administered over a short infusion time. The infusion given at any dose level should be dependent upon the toxicity achieved after each. Hence, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade lll or lV toxicity in any category. Doses that are 2/3 of this value would be defined as the safe dose.

Physical examination, tumor measurements, and laboratory tests should, of course, be performed before treatment and at intervals of about 3-4 weeks later. Laboratory tests should include complete blood counts, serum creatinine, creatine kinase, electrolytes, urea, nitrogen, SGOT, bilirubin, albumin, and total serum protein.

Clinical responses may be defined by acceptable measure or changes in laboratory values e.g. tumormarkers. For example; a complete response may be defined by the disappearance of all measurable disease for at least a month. Whereas a partial response may be defined by a 50% or greater reduction.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### Administration and dosing

The present invention includes a method of delivery of a pharmaceutically effective amount of the inventive formulation of a low molecular weight compound to an angiogenic vascular target site of a subject in need thereof. A "subject in need thereof" thereby refers to a mammal, e. g. a human.

The route of administration comprises peritoneal, parenteral or topic administration and the formulations are easily administered in a variety of dosage forms such as injectable solutions, drug release capsules and the like.
For use with the present invention the term "pharmacologically effective amount" of a compound administered to a subject in need thereof (which may be any animal with a circulatory system with endothelial cells which undergo angiogenesis) will vary depending on a wide range of factors. For example, it would be necessary to provide substantially larger doses to humans than to smaller animal. The amount of the compound will depend upon the size, age, sex, weight, and condition of the patient as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which target the surrounding tissue e. g., target the tumor cells themselves.
The pharmaceutically effective amount of a therapeutic agent as disclosed herein depends on the kind and the type of action of the agent. For the examples mentioned herein, it is within the range of about about 0.1 to 20 mg/kg in humans. Typically, for camptothecin drugs, doses in the order of about 5 mg/kg are applied.

### Figure captions

- Fig. 1:: Equilibrium between camptothecin in the lactone form (left) and the carboxylate form (right). The equilibrium is pH dependent and reversible. At pH values close to 7.5, the carboxylate form predominates.
- Fig 2:: One reason for the enhanced cytotoxicity of the CPT-carboxylate is its reduced membrane permeability due to the negative charge. Therefore the delivery to the target site is hindered. On the other hand, in organs, where a more acidic pH is present, the CPT may reorganize into the lactone form and then it may penetrate more easily across the cell membrane, thus inducing the toxic side effects.
- Fig. 3: Equilibrium between the carboxylate, the lactone and the cationic molecule-bound form of CPT
- Fig. 6:: Liposomal organization of CPT in the lactone form (left) and in the lipid-bound form (right). Only a limited amount of the lactone can be embedded in the hydrophobic compartment of the liposome. Due to the amphiphilic properties of CM-CPT, it can act as an integral part of the membrane and much higher drug/lipid ratios can be obtained.
- Fig. 7:: Occurrences at the plasma membrane. The binding of the CPT to the lipid makes it more amphiphilic itself. Therefore, processes as insertion into the plasma membrane after binding and/or fusion, and permeation across the membrane are facilitated. The pH shift on endocytosis favours the release of the CPT in the lactone form.
- Fig. 4: Excitation and fluorescence spectra of camptothecin, dissolved in Chloroform/Methanol and in the liposomal form.
- Fig.5: UV-vis spectra of CPT under different conditions. The spectrum of the liposomal CPT (top curve) differs clearly from those under all other conditions. Characteristic shapes can be made out for the liposomal CPT, the CPT-carboxylate (second and third curve from above) and the lactone from of the CPT (fourth and fifth curve from above). The lactone spectrum shows as well a strong dependence on the solvent environment. For clarity the spectra are vertically shifted.
- Fig. 8:: Dialysis of lipid suspensions together with CPT-carboxylate present in the solution. The solutions were 10 mM in lipid, 1 mM CPT-carboxylate, pH 7.5. With DOPC virtually all CPT is released into the dialysate, while with DOTAP a substantial retention effect is determined.
- Fig. 9: Fluorescence anisotropy measurements of CPT in lipid suspensions from DOTAP and DOPC and as prepared by different methods. The measurements were performed with 10 mM Tris-buffer, pH, 7.5 and with 10 % trehalose present in the aqueous phase. For DOPC the standard film method was used (second column). The third column shows the results for liposome formation from DOTAP and CPT in the lacton form by the film method and the third column shows the results when CPT-carboxylate was used instead of the lactone. The fifth column shows ethanol injection into aqueous solution of CPT-carboxylate. Only with DOTAP a substantial anisotropy was detected. The value was highest with the ethanol injection procedure, indication that in this case as well the binding efficacy was highest.
- Fig. 10:: Time dependence of fluorescence anisotropy after formulation for DOTAP/CPT and DOPC/CPT liposomes by the film method using CPT in the lactone form. The measurements were performed with 10 mM Tris-buffer, pH, 7.5 and with 10 % trehalose present in the aqueous phase. For DOPC the anisotropy remains at a low value, while with DOTAP the anisotropy is much higher and it increases significantly after formulation. It is expected that the increase in anisotropy is due to a rearrangement of the CPT form the lactone to the carboxylate and subsequent efficient binding to the DOTAP.
- Fig. 11:: Inhibition of Ea.Hy 926 growth by Camptothecin formulations RM541, 542, 543, 544. For details see text.
- Fig. 12:: Therapeutic efficacy of LipoCam in A-375 melanoma of nude mice. For details see text.
- Tab. 1:: Results from physico-chemical characterization of frequently used liposomal formulations
- Tab. 2:: Results from size measurements of liposomal suspensions of DOTAP comprising CPT. Measurements after extrusion (200 nm pore size), after diafiltration (to remove free CPT-carboxylate) and after lyophilization, storage and reconstitution to the original concentration are given. The results demonstrate, that, under suitable conditions, the suspensions can be lyophilised for storage and reconstituted without affecting the physical state of the liposomes.
- Tab. 3:: Formulations and dosages for the animal studies

**Tab. 1**

| Formulation | Composition DOTAP/DOPC/CPT | Total molarity [mM] | Size [nm] | Pl | Zeta potential |
|---|---|---|---|---|---|
| | | | | | |
| Lipocam I | 55/40/5 | 10 | 189.4 | 0.116 | 54 |
| | | 15 | | | |
| | | 25 | | | |
| Lipocam II | 60/30/10 | 10 | 179 | 0.146 | 57 |
| | | 15 | | | |
| | | 25 | | | |
| Lipocam lll | 90/10 | 10 | 194 | 0.131 | 65 |
| | | 15 | | | |
| | | 25 | | | |

**Tab. 2**

| Formulation | Extrusion Zₐᵥₑ [nm] Pl | Diafiltration Zₐᵥₑ [nm] Pl | Lyophilization Zₐᵥₑ [nm] Pl |
|---|---|---|---|
| RM622 | 196 | 195 | 193 |
| DOTAP/CPT 90/10 | 0.082 | 0.096 | 0.112 |
| 10 mM | | | |
| | | | |
| RM623 | 191 | 204 | 203 |
| DOTAP/CPT 90/10 | 0.179 | 0.207 | 0.2 |
| 15 mM | | | |
| RM624 | 207 | 211 | 258 |
| DOTAP/CPT 90/10 | 0.409 | 0.4 | 0.545 |
| 20 mM | | | |
| RM625 | 174 | 189 | 258 |
| DOTAP/CPT 90/10 | 0.101 | 0.255 | 0.566 |
| 25 mM | | | |
| RM626 | 203 | 201 | 193 |
| DOTAP/CPT 95/5 | 0.189 | 0.191 | 0.173 |
| 15 mM | | | |
| RM627 | 163 | 191 | 179 |
| DOTAP/CPT 85/15 | 0.234 | 0.336 | 0.219 |
| 15 mM | | | |
| RM628 | 170 | 195 | 169 |
| DOTAP/CPT 80/20 | 0.188 | 0.294 | 0.27 |
| 15 mM | | | |

**Tab. 3**

| Sample | | ^{Z}average Pl | | ^{Z}average Pl |
|---|---|---|---|---|
| DOTAP/CPT 90/10 | After extrusion | | Concentrated to 7% of original volume, and then brought back to the previous conentration | 166 |
| 15 mM | | 167 | | 0.24 |
| 1 % Trehalose | | 0.14 | | |
| DOTAP/CPT 85/15 | After extrusion | 180 | Concentrated to 7% of original volume, and then brought back to the previous conentration | 160 |
| 15 mM | | 0.12 | | 0.16 |
| 1 % Trehalose | | | | |
| 1. Adding of 3mM DOTAP in Ethanol to 1mM CPT-carboxylate in H₂O. (Drug/lipid ratio 1/9) | Final Concentration = 10 mM | 111 | Final Concentration = 20 mM | 67.6 |
| | | 0.428 | | 0.342 |
| | | | | |
| 2. Solvent evaporation | | | | |
| 1. Adding of 6 mM | Dilution from 100mM to 10mM | 93 | | |
| DOTAP in Ethanol to 2 mM CPT-carboxylate in H₂O. (Drug/lipid ratio 1/9) | | 0.619 | | |
| 2. Solvent evaporation. Final concentration 100 mM and 500 mM | | | | |

**Tab. 4**

| Group | Drug | Dose [mg/kg] | N° of mice |
|---|---|---|---|
| 0 | 5 % Glucose | / | 8 |
| 1 | LipoCam I | 5 | 8 |
| 2 | LipoCam II | 5 | 8 |
| 3 | Lipocam lll | 2,5 | 8 |
| 4 | CPT-Na-Carboxylate | 1,0 | 8 |

## Claims

1. A **composition** comprising the carboxylate form of a camptothecin drug or a derivative thereof associated with at least one organic cationic molecule which has a positive net charge(cationic molecule), wherein said composition has a molar ratio of at least about 1:1 of an organic cationic molecule to camptothecin carboxylate and is substantially free of the lactone form of said drug or a derivative thereof.

2. The composition of claim 1, wherein said camptothecin carboxylate is selected from the ammonium, sodium or potassium salt of a camptothecin drug or a derivative thereof.

3. The composition of claim 1 to 2 wherein said organic cationic molecule is an amphiphile or a polymer.

4. The composition of any one of claims 1 to 3, wherein said cationic amphiphile is selected from lipids, lysolipids or pegylated lipids, preferably a cationic amphiphile with a tertiary amino or quaternary ammonium group such as N-[1-(2,3-diacyloxy)propyl]-N,N-dimethylamine or N-[1-(2,3-diacyloxy)propyl]-N,N,N-trimethyl ammonium.

5. The composition of any one of claims 1 to 4, wherein said polymer is a polyelectrolyte, acid such as polyallylamine or polyethylene imine, a polymeric sugar or a polyamino with a molecular weight between about 5 and 500 kDa.

6. The composition of any one of the claims 1 to 5, further comprising at least one amphiphile which has a negative and/or neutral net charge (anionic and/or neutral amphiphile).

7. The composition of any one of claims 1 to 6, wherein said anionic and/or neutral amphiphile is selected from sterols or lipids such as cholesterol, phospholipids, lysolipids, lysophospholipids, sphingolipids or pegylated lipids with a negative or neutral net change.

8. The composition of any one of the claims 1 to 7, wherein the neutral amphiphile is diacylphosphatidylcholine.

9. **A colloidal nanoaggregate** comprising a composition of any one of the claims 1 to 8.

10. The nanoaggregate of claim 9 having an overall positive charge.

11. The nanoaggregate of claim 9 or 10, comprising an excess of positively charged moieties of at least 20 %, preferably at least 30 % and most preferably at least 40 % in the outer molecular layer.

12. The nanoaggregate of any one of the claims 9 to 11, which is present as an emulsion droplet, a micelle, a liposome, a nanoparticle or a nanocapsule.

13. The nanoaggregate of any one of the claims 9 to 12, comprising about 1 to 50 mol % of the drug, preferably about 5 to 15 mol % of the drug.

14. The nanoaggregate of any one of the claims 9 to 13 which is a particle having a particle size ranging from about 5 nm to 5000 nm, preferably from 25 nm to 500 nm and more preferably from about 100 nm to 300 nm.

15. The nanoaggregate of any one of the claims 9 to 14, further comprising a cryoprotectant which is selected from a sugar or an alcohol or a combination thereof such as trehalose, maltose, sucrose, glucose, lactose, dextran, mannitol or sorbitol.

16. **A pharmaceutical preparation** comprising a pharmaceutically effective amount of the composition of any one of the claims 1 to 8 or a colloidal nanoaggregate of any one of the claims 9 to 15 together with a pharmaceutically acceptable carrier, diluent and/or adjuvant.

17. **A method of producing** the colloidal nanoaggregate of any one of the claims 9 to 15, comprising the steps of
a) providing a camptothecin drug or derivative thereof, preferably as a salt and
b) associating said drug with a cationic amphiphile which has a positive net charge (cationic amphiphile)
c) and optionally at least one further amphiphile which has a positive, negative and/or neutral net charge (anionic and/or neutral amphiphile) forming a colloidal nanoaggregate.

18. The method of claim 17, wherein step b) and c) comprise forming said nanoaggregate by a homogenisation, a lipid film or by a solvent injection procedure.

19. **The use of** a pharmaceutical preparation of claim 16 for producing a medicament for treating and/or preventing a disease **characterized by** enhanced angiogenic activity.
